# EUROPÄISCHE PATENTSCHRIFT

(11) **EP 2 566 382 B1**
(45) Veröffentlichungstag und Bekanntmachung des Hinweises auf die Patenterteilung: **14.09.2016**
(21) Anmeldenummer: 11718981.1
(22) Anmeldetag: 05.05.2011
(51) Int. Cl.: A61B 5/00, B31D 1/00

(54) **VERFAHREN ZUR HERSTELLUNG EINES MEMBRANRINGS ODER TESTSTREIFENRINGS UND RINGMAGAZIN**
METHOD FOR PRODUCING A MEMBRANE RING OR TEST STRIP RING AND RING MAGAZINE
PROCÉDÉ DE PRODUCTION D'UNE MEMBRANE ANNULAIRE OU D'UNE BANDELETTE DE TEST ANNULAIRE ET MAGASIN APPROPRIÉ

(30) Priorität: 05.05.2010 EP 10162064
(43) Veröffentlichungstag der Anmeldung: 13.03.2013
(73) Patentinhaber: Roche Diabetes Care GmbH, 68305 Mannheim (DE); F.Hoffmann-La Roche AG, 4070 Basel (CH)
(72) Erfinder: HARTTIG, Herbert, 67434 Neustadt (DE)
(74) Vertreter: Pfiz, Thomas
(86) Internationale Anmeldenummer: PCT/EP2011/057193
(87) Internationale Veröffentlichungsnummer: WO 2011/138388

(56) Entgegenhaltungen:
- EP-A1- 0 756 101
- WO-A1-2009/037192
- US-A1- 2006 157 362

## Beschreibung

Die Erfindung betrifft ein Verfahren zur Herstellung eines Membranrings oder Teststreifenrings für eine diagnostische Testeinrichtung insbesondere zur Untersuchung von Körperflüssigkeiten. Die Erfindung betrifft weiter ein Ringmagazin als Verfahrensprodukt für eine diagnostische Testeinrichtung.

Im Bereich der medizinischen Diagnostik ist der Einsatz von Mikrofiltrationsmembranen in Form von Streifen oder Scheiben an sich bekannt. Auch die Verwendung von scheibenförmigen Magazinen in Handgeräten für Blutzuckermessungen ist beispielsweise aus der WO2009/037192 bekannt. Nachteilig beim direkten Zuschnitt von Flachmaterialringen ist der Verlust an Material, der besonders bei schmalen Ringen sehr ausgeprägt ist. So werden bei einem Ring von 49 mm Außendurchmesser und 39 mm Innendurchmesser von einer quadratischen Ausgangsfläche, aus der der Ring ausgeschnitten wird, nur 28,8% genutzt. Werden die Ringe in dichtest möglicher Anordnung aus einer Ausgangsfläche geschnitten, so können theoretisch maximal 33,2% genutzt werden, praktisch weniger. Bei den hohen Kosten von Membranen spielt die Ausbeute an nutzbarer Fläche vor allem für Massenprodukte eine wichtige ökonomische Rolle. Zudem ist die Handhabung von einzelnen Membranflächen zeitlich und mechanisch aufwändig und fehleranfällig.

Aus der EP-A 0 756 101 ist ein Verfahren zum Herstellen eines ebenen Reibringes bekannt, der als Reibbelag auf einen stählernen Tragring aufgeklebt wird. Dort sind die Einschnitte winklig ausgeführt, so dass die in dem Ring gebildeten Ölnuten durch parallel zueinander verlaufende Schnittkanten begrenzt sind.

Die US-A 2006/0157362 offenbart eine Testkassette mit gesonderten, radial ausgerichteten Teststreifen, die durch Sektoren voneinander getrennt sind.

Ausgehend hiervon liegt der Erfindung die Aufgabe zugrunde, die im Stand der Technik bekannten Verfahren und Vorrichtungen weiter zu verbessern und im Bereich diagnostischer Anwendungen den Materialeinsatz und die Handhabung von Verbrauchsmitteln zu optimieren.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, für eine Mehrzahl von Einzeltests in einer ringförmigen Anordnung jeweils ein analytisches Materialsegment insbesondere als Magazin bereitzustellen. Dementsprechend wird in verfahrensmäßiger Hinsicht vorgeschlagen, dass
a) ein vorzugsweise durch Zuschneiden eines flächigen oder bandförmigen Ausgangsmaterials bereitgestellter langgestreckter Streifen durch quer (d.h. rechtwinklig oder schräg) zur Streifenlängsrichtung verlaufende Einschnitte in Segmente unterteilt wird,
b) wobei die Einschnitte nur bis zu einer ggf. variierenden Restbreite des Streifens geführt werden, so dass zwischen den an die Einschnitte angrenzenden Segmenten eine Materialbrücke erhalten bleibt;
c) der Streifen unter Zusammenführen seiner Enden zu einem Ring geschlossen wird, wobei die zu den Materialbrücken hin verlaufenden Schnittkanten der Einschnitte jeweils einen spitzen Winkel einschließen;
d) der Ring als Membranring oder Teststreifenring für die Testeinrichtung in einer Tragstruktur eingesetzt wird.

Auf diese Weise kann das direkte Ausstanzen eines Materialrings vermieden und das zu verwerfende Material während des Herstellungsprozesses minimiert werden. Außerdem kann die Automatisierung durch eine lineare Vorfertigung beispielsweise von Rolle zu Rolle vereinfacht werden. Zugleich wird durch die Segmentierung eine Aufteilung in Einzeltests besonders vereinfacht.

Gemäß einer bevorzugten Ausgestaltung sollte der Streifen bis auf eine als Materialbrücke verbleibende Restbreite von 0,1 bis 1,0 mm eingeschnitten werden, um eine hinreichende Biegsamkeit zu gewährleisten. Bevorzugt wird eine einheitliche Restbreite gewählt.

Eine vorteilhafte Ausführung sieht vor, dass der Streifen einheitlich von einem seiner Längsränder her eingeschnitten wird, so dass die Materialbrücken an dem anderen Längsrand verbleiben. Liegen die Materialbrücken am Außenrand des nachfolgend gebildeten Rings, ist die Überlappung der Segmente maximal, während bei Materialbrücken am Innenrand keine Überlappung erfolgt.

Gemäß einer weiteren vorteilhaften Variante wird der Streifen auf jeweils einer Linie von seinen beiden Längsrändern her unter Erhaltung einer Materialbrücke beidseitig eingeschnitten.

Die Einschnitte lassen sich vorteilhafterweise mit einem Schneid- oder Stanzwerkzeug oder durch Laserschneiden erzeugen. Der Schnitt mit einem Laser verdichtet die Schnittkante und stabilisiert dadurch die Materialbrücke. Daher wird ein Laserschnitt bevorzugt.

Herstellungstechnisch ist es von Vorteil, wenn der eingeschnittene Streifen vor der Ringbildung in seiner Längsrichtung tordiert wird, so dass die Segmente bezüglich einer durch die Materialbrücken verlaufenden Kippachse mit fortlaufend zunehmendem Winkel gekippt sind. Dadurch ist eine nachfolgen-de Ringformung möglich, ohne dass die Schnittkanten aneinander verhaken.

Vorteilhafterweise werden die Segmente bei der Ringbildung gemäß Schritt c) sukzessive auf einer ebenen Ringfläche abgelegt und dabei aus einer Kippstellung in eine gegenseitige Überlappstellung gebracht.

In dem vorgenannten Verfahrensschritt c) wird der Ring vorteilhafterweise so geformt, dass die Materialbrücken eine Kreislinie aufspannen, wobei die Enden des Streifens sich auf der Kreislinie kontaktieren.

Eine weitere Verbesserung des Verfahrensproduktes lässt sich dadurch erzielen, dass die Segmente bei der Ringbildung paarweise überlappend aufeinander abgelegt werden, wobei die durch Schnittkanten der Einschnitte begrenzten Überlappungen von der Ringinnenseite zu der jeweiligen Materialbrücke hin verlaufen.

Um den gebildeten Ring zu stabilisieren, ist es vorteilhaft, wenn benachbarte Segmente vorzugsweise unter Einwirkung von Wärme und/oder Druck überlappend miteinander verbunden, insbesondere verschweißt oder verklebt werden. Günstig ist es auch, wenn bei einer gegenseitigen Verbindung der Segmente zugleich eine Fixierung des Rings auf der Tragstruktur, bevorzugt in einem Magazin erfolgt.

Ein besonders bevorzugter Einsatz sieht ein Ausgangsmaterial vor, das durch eine mit einer reaktiven Testchemie beschichtete Folie oder durch eine für die Körperflüssigkeit zumindest partiell durchlässige Membran gebildet wird. Vorteilhaft kann ein Membranring auf einen Teststreifenring unter paarweisem Kontakt der beiderseitigen Segmente aufgebracht werden. Denkbar ist es auch, dass einzelne Testfelder auf die Segmente eines Membranrings aufgebracht werden, oder umgekehrt.

In Zusammenhang mit diagnostischen Anwendungen ist es von Vorteil, wenn die Segmente als Verbrauchsmittel in einem Ringmagazin bevorzugt in Kammern getrennt für jeweils einen Einzeltest bereitgestellt bzw. angeordnet werden. Bevorzugt werden solche Kammern mit jeweils einem Stech- bzw. Nadelelement zur Gewinnung von Körperflüssigkeit bestückt.

Vorteilhafterweise wird der geschlossene Ring in ein scheibenförmiges Gehäuse als Tragstruktur eingelegt, so dass die Segmente jeweils einer Kammer des Gehäuses zugeordnet sind.

Eine weitere vorteilhafter Ausgestaltung sieht vor, dass der Streifen von einem Längsrand her rechtwinklig eingeschnitten wird, wobei die Schnittkanten der so gebildeten Einschnitte jeweils paarweise parallel zu einer gemeinsamen Schnittlinie verlaufen.

Um einen Teststreifenring zu realisieren, kann eine mit einer reaktiven Test-chemie für einen Analyten in der Körperflüssigkeit beschichtete Folie als Ausgangsmaterial verwendet werden. Für einen Membranring zur selektiven Separation von Inhaltsstoffen der Körperflüssigkeit ist es vorteilhaft, als Ausgangsmaterial eine Filtermembran einzusetzen.

Gegenstand der Erfindung ist auch ein Ringmagazin für eine diagnostische Testeinrichtung insbesondere zur Untersuchung von Körperflüssigkeiten mit einer Tragstruktur und einem darin eingesetzten Membranring oder Teststreifenring hergestellt nach dem erfindungsgemäßen Verfahren, wobei der als umlaufendes Gebilde geschlossen eingesetzte Ring aus einem durch Einschnitte segmentierten Streifenmaterial gebildet ist und die über Materialbrücken verbundenen Segmente ein Filter oder ein Nachweiselement für jeweils einen Einzeltest bilden. Ein solcher Membranring oder Teststreifenring weist demnach einen Streifen auf, der durch Randeinschnitte quer zur Streifen-längsrichtung in Segmente unterteilt ist, wobei die Einschnitte nur bis zu einer ggf. variierenden Restbreite des Streifens verlaufen, so dass zwischen den an die Einschnitte angrenzenden Segmenten eine Materialbrücke erhalten bleibt, und wobei der Streifen zu einem Ring geschlossen ist, so dass die bis zu den Materialbrücken hin verlaufenden Schnittkanten der Einschnitte jeweils einen spitzen Winkel einschließen.

Bei einem solchen Ringmagazin kann die Tragstruktur Öffnungen bzw. Fenster für den Transfer von Körperflüssigkeit aufweisen, wobei die Öffnungen von jeweils einem Segment des Membranrings oder Teststreifenrings abge-deckt sind. Bevorzugt weist die Tragstruktur in einem scheibenförmigen Gehäuse ringförmig angeordnete Testkammern auf, die vorteilhafterweise jeweils ein Stechelement enthalten, und die jeweils einem Segment des Membran- oder Teststreifenrings für die Durchführung eines Einzeltests zugeordnet sind. Hierbei sollten die Segmente jeweils eine freie Applikationsstelle zur Aufnahme von Körperflüssigkeit aufweisen.

Ein weiterer Erfindungsaspekt betrifft eine diagnostische Testeinrichtung, die zur sukzessiven segmentweisen Verarbeitung eines erfindungsgemäßen Ringmagazins eingerichtet ist. Insbesondere kann in einem solchen Instrument das auswechselbare Ringmagazin drehbar gelagert bzw. durch einen Drehantrieb rotierbar sein, um die einzelnen Segmente an einer definierten Applikationsstelle bereitzustellen.

Im Folgenden wird die Erfindung anhand eines in der Zeichnung schematisch dargestellten Ausführungsbeispiels näher erläutert. Es zeigen
- Fig. 1: ein Ringmagazin für ein diagnostisches Testgerät mit einem nur hälftig gezeigten Teststreifenring in perspektivischer Darstellung;
- Fig. 2: eine schaubildliche Darstellung einer Vorrichtung zur Vorfertigung eines eingeschnittenen Teststreifens;
- Fig. 3 bis 5: verschiedene Stufen einer materialsparenden Bildung des Teststreifenrings aus dem vorgefertigten Teststreifen in abgebrochener Draufsicht.

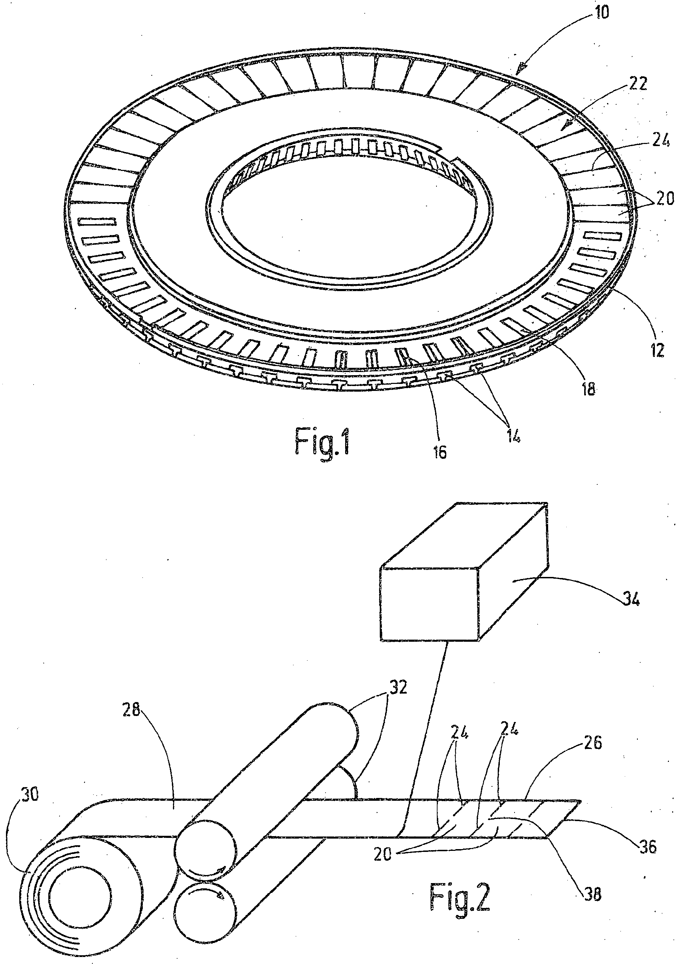

Das in Fig. 1 dargestellte Ringmagazin lässt sich als Verbrauchsmaterial in ein nicht gezeigtes mobiles Analysegerät für Blutzuckeranalyen einsetzen. Es umfasst zu diesem Zweck ein scheibenförmiges Gehäuse 12 als Tragstruktur, in welchem eine Vielzahl von kreisförmig angeordneten Kammern 14 zur Aufnahme von radial ausschiebbaren Stechelementen 16 enthalten sind, wobei auf dem Gehäusedeckel über den Kammern 12 jeweils eine Öffnung 18 vorgesehen ist, über die bei einem Körpereinstich gewonnenes Blut für einen photometrischen Glukosenachweis auf ein Segment 20 eines Teststreifenrings 22 übertragen werden kann. Der Teststreifenring kann mit einer trockenchemischen Reagenzschicht versehen sein, welche die applizierte Körperflüssigkeit aufnimmt und mit einem darin enthaltenen Analyten, beispielsweise Glukose reagiert. Denkbar ist es auch, dass ein Membranring als Filter zum Blutübertrag zwischen den Stechelementen 16 und einem Testring angebracht wird.

Um einen solchen Teststreifen- bzw. Membranring 22 möglichst materialsparend herstellen zu können, ist eine Ringfertigung aus einem durch Einschnitte 24 segmentierten Streifenmaterial vorgesehen, wie es nachstehend näher erläutert wird. Damit kann das direkte Ausstanzen eines Flachmaterialrings vermieden und das zu verwerfende Material während des Herstellungsprozesses minimiert werden.

Fig. 2 veranschaulicht die Vorfertigung eines eingeschnittenen Bandstreifens 26. Dabei wird ein Band 28 als Ausgangsmaterial von einer Vorratsspule 30 abgezogen und über Transportwalzen 32 geführt. Ein Laser 34 als Schneidgerät ermöglicht mittels Laserstrahl 36 das Ablängen des Streifens 26 an Streifenenden 36 und das Einbringen der Einschnitte 24 unter Bildung der Segmente 20 in dem Bandmaterial. Alternativ ist es auch denkbar, von einem großflächigen Ausgangsmaterial zunächst Streifen abzuschneiden und diese dann mit den Quereinschnitten zu versehen.

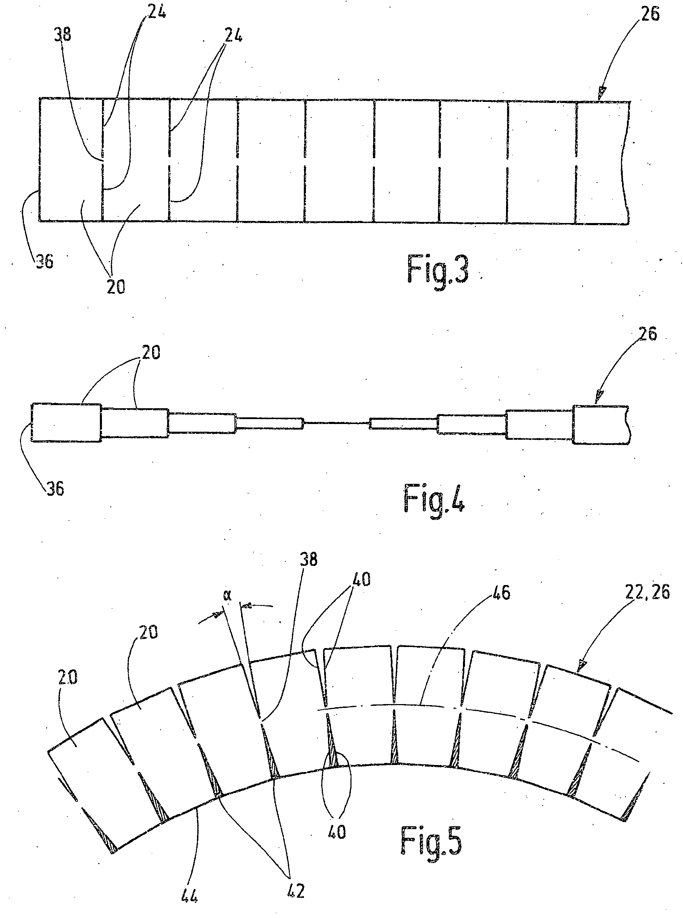

Wie auch aus Fig. 3 ersichtlich, ist bei dem gezeigten Ausführungsbeispiel der Streifen 26 auf jeweils einer Querlinie rechtwinklig zu den Längsrändern beidseitig eingeschnitten, so dass eine zentrale Materialbrücke 38 zwischen benachbarten Segmenten 20 erhalten bleibt. Möglich ist es auch, dass der Streifen einheitlich von einem Längs- bzw. Seitenrand her rechtwinklig eingeschnitten wird, so dass die Materialbrücken dann an dem gegenüberliegenden anderen Längsrand angeordnet sind. Zweckmäßig liegen die Einschnitte 24 in Längsrichtung des Streifens 26 gesehen in gleichem Abstand voneinander, um deckungsgleiche Segmente 20 zu erhalten.

Beispielsweise kann ein Bandmaterial von 90 µm Dicke in einen Streifen mit einer Länge von 140 mm und einer Breite von 5 mm geschnitten werden, während die Einschnitte im Längsabstand von 2,8 mm liegen und von beiden Längsrändern aus jeweils 2,4 mm weit quer verlaufen, so dass eine Materialbrücke von 0,2 mm verbleibt.

Wie in Fig. 4 gezeigt, wird der eingeschnittene Streifen 26 vor der Ringbildung in seiner Längsrichtung tordiert, so dass die Segmente 20 bezüglich einer durch die Materialbrücken 38 verlaufenden Längsachse fortlaufend mit zunehmendem Winkel gekippt bzw. verdreht sind und entsprechend in der Draufsicht unterschiedlich breit erscheinen. Durch diese Maßnahme wird eine kollisionsfreie Ringbildung mit gegenseitigem Überlapp der Segmente 20 ermöglicht.

Fig. 5 illustriert die Ausbildung des Ringes 22 aus dem vorgefertigten Streifen 26 gemäß Fig. 3 und 4. Dabei werden die Segmente 20 sukzessive auf einer ebenen Ringfläche, zweckmäßig direkt auf dem Gehäusedeckel abgelegt. Beim Ablegen wird die Kippstellung der Segmente 20 in eine gegenseitige Überlappstellung zurückgeführt. Die durch die einander gegenüberliegenden Schnittkanten 40 der Einschnitte 24 begrenzten Überlappbereiche 42 verlaufen dann von der Ringinnenseite 44 bis zu der jeweiligen Materialbrücke 38 hin, während die radial äußeren Schnittkanten 40 auch unter einem spitzen Winkel α von der jeweiligen Materialbrücke 38 ausgehend auseinanderklaffen. In dem so gebildeten Ring 26 spannen die Materialbrücken eine Kreislinie 46 auf, wobei die beiden Streifenenden 36 sich auf der Kreislinie kontaktieren.

Zweckmäßig werden die Segmente 20 in den Überlappbereichen 42 durch Einwirkung von Wärme und Druck miteinander verschweißt, beispielsweise mittels eines entsprechend strukturierten Heizstempels oder mittels eines transparenten Stempels und Laserlicht. Dabei kann zugleich eine stoffschlüssige Fixierung auf dem Gehäusedeckel erreicht werden.

## Patentansprüche

1. Verfahren zur Herstellung eines Membranrings oder Teststreifenrings für eine diagnostische Testeinrichtung insbesondere zur Untersuchung von Körperflüssigkeiten bei dem
a) ein vorzugsweise durch Zuschneiden eines flächigen oder bandförmigen Ausgangsmaterials bereitgestellter langgestreckter Streifen (26) durch quer zur Streifenlängsrichtung verlaufende Einschnitte (24) in Segmente (20) unterteilt wird,
b) wobei die Einschnitte (24) nur bis zu einer Restbreite des Streifens (26) geführt werden, so dass zwischen den an die Einschnitte (24) angrenzenden Segmenten (20) eine Materialbrücke (38) erhalten bleibt;
c) der Streifen (26) unter Zusammenführen seiner Enden zu einem Ring (22) geschlossen wird,
**dadurch gekennzeichnet, dass**
d) die zu den Materialbrücken (38) hin verlaufenden Schnittkanten der Einschnitte (24) jeweils einen spitzen Winkel (α) einschließen;
e) der Ring (22) als Membranring oder Teststreifenring für die Testeinrichtung in einer Tragstruktur (12) eingesetzt wird, wobei der Ring (22) in ein scheibenförmiges Gehäuse (12) als Tragstruktur eingelegt wird, so dass die Segmente (20) jeweils einer Kammer (14) des Gehäuses zugeordnet sind.

2. Verfahren nach Anspruch 1, **dadurch gekennzeichnet, dass** der Streifen (26) einheitlich von einem seiner Längsränder her eingeschnitten wird, so dass die Materialbrücken (38) an dem anderen Längsrand verbleiben.

3. Verfahren nach Anspruch 1, **dadurch gekennzeichnet, dass** der Streifen (26) auf jeweils einer Linie von seinen beiden Längsrändern her unter Erhaltung einer Materialbrücke (38) beidseitig eingeschnitten wird.

4. Verfahren nach einem der Ansprüche 1 bis 3, **dadurch gekennzeichnet, dass** der Ring (22) derart geformt wird, dass die Materialbrücken (38) eine Kreislinie (46) aufspannen, wobei die Enden des Streifens (26) sich auf der Kreislinie kontaktieren.

5. Verfahren nach einem der Ansprüche 1 bis 4, **dadurch gekennzeichnet, dass** die Segmente (20) bei der Ringbildung paarweise überlappend aufeinander abgelegt werden, wobei die durch Schnittkanten der Einschnitte (24) begrenzten Überlappungen (42) von der Ringinnenseite zu der jeweiligen Materialbrücke (38) hin verlaufen.

6. Verfahren nach einem der Ansprüche 1 bis 5, **dadurch gekennzeichnet, dass** benachbarte Segmente (20) vorzugsweise unter Einwirkung von Wärme und/oder Druck überlappend miteinander verbunden, insbesondere verschweißt oder verklebt werden.

7. Verfahren nach einem der Ansprüche 1 bis 6, **dadurch gekennzeichnet, dass** das Ausgangsmaterial durch eine mit einer reaktiven Testchemie beschichtete Folie oder durch eine für die Körperflüssigkeit zumindest partiell durchlässige Membran gebildet wird.

8. Verfahren nach einem der Ansprüche 1 bis 7, **dadurch gekennzeichnet, dass** die Segmente (20) als Verbrauchsmittel in einem Ringmagazin (10) für jeweils einen Einzeltest angeordnet werden.

9. Verfahren nach einem der Ansprüche 1 bis 8, **dadurch gekennzeichnet, dass** als Ausgangsmaterial eine zur Filterung der Körperflüssigkeit ausgebildete Membran oder eine mit einer reaktiven Testchemie für einen Analyten in der Körperflüssigkeit beschichtete Folie verwendet wird.

10. Ringmagazin für eine diagnostische Testeinrichtung insbesondere zur Untersuchung von Körperflüssigkeiten mit einer Tragstruktur (12) und einem darin eingesetzten, aus einem durch Einschnitte (24) segmentierten Streifenmaterial gebildeten geschlossenen Membranring oder Teststreifenring, **dadurch gekennzeichnet, dass** der Membranring oder Teststreifenring (22) nach einem der vorhergehenden Ansprüche hergestellt ist und die Segmente (20) ein Filter oder ein Nachweiselement für jeweils einen Einzeltest bilden.

11. Ringmagazin nach Anspruch 10, **dadurch gekennzeichnet,** das die Tragstruktur (12) eine Mehrzahl von Öffnungen (18) für den Transfer von Körperflüssigkeit aufweist, und dass die Öffnungen (18) von jeweils einem Segment (20) des Membranrings oder Teststreifenrings (22) abgedeckt sind.

12. Ringmagazin nach Anspruch 10 oder 11, **dadurch gekennzeichnet, dass** die Tragstruktur (12) ein scheibenförmiges Gehäuse (12) mit ringförmig angeordneten, jeweils einem Segment (20) zugeordneten Testkammern aufweist.

13. Ringmagazin nach einem der Ansprüche 10 bis 12, **dadurch gekennzeichnet, dass** die Segmente (20) jeweils eine Applikationsstelle zur Aufnahme von Körperflüssigkeit aufweisen.

14. Ringmagazin nach einem der Ansprüche 10 bis 13, **dadurch gekennzeichnet, dass** der Membranring (22) als Filter einem ringförmig ausgebildeten Nachweiselement vorgeordnet ist.

15. Diagnostische Testeinrichtung zur Untersuchung von Körperflüssigkeiten mit einem Gehäuse und einem darin eingesetzten Ringmagazin nach einem der Ansprüche 10 bis 14.

## Claims

1. Process for producing a membrane ring or test strip ring for a diagnostic test device in particular for examining body fluids in which
a) an elongate strip (26) preferably provided by cutting a flat or tape-shaped starting material is divided into segments (20) by cuts (24) running transversely to the longitudinal direction of the strip,
b) wherein the cuts (24) are made only as far as a residual width of the strip (26) so that a material bridge (38) remains intact between the segments (20) adjacent to the cuts (24);
c) the strip (26) is closed by bringing its ends together to form a ring (22),
**characterized in that**
d) the cut edges of each **of** the cuts (24) running towards the material bridges (38) enclose an acute angle (α);
e) the ring (22) is inserted into a support structure (12) as a membrane ring or test strip ring for the test device, wherein the ring (22) is inserted into a disc-shaped housing (12) forming a support structure so that each of the segments (20) is allocated to one chamber (14) of the housing.

2. Process according to claim 1, **characterized in that** the strip (26) is uniformly cut into from one of its longitudinal edges such that the material bridges (38) remain at the other longitudinal edge.

3. Process according to claim 1, **characterized in that** the strip (26) is cut two-sided in each case on one line from both of its longitudinal edges while retaining a material bridge (38).

4. Process according to one of the claims 1 to 3, **characterized in that** the ring (22) is formed in such a manner that the material bridges (38) span a circle (46) wherein the ends of the strip (26) make contact on the circle.

5. Process according to one of the claims 1 to 4, **characterized in that** the segments (20) are put down overlapping in pairs during ring formation, wherein the overlaps (42) bounded by the cut edges of the cuts (24) run from the inner side of the ring to the respective material bridge (38).

6. Process according to one of the claims 1 to 5, **characterized in that** neighbouring segments (20) are joined to one another in an overlapping manner preferably under the action of heat and/or pressure, in particular welded or glued.

7. Process according to one of the claims 1 to 6, **characterized in that** the starting material is formed by a foil coated with a reactive test chemistry or by a membrane that is at least partially permeable to the body fluid.

8. Process according to one of the claims 1 to 7, **characterized in that** the segments (20) are arranged as disposables in a ring magazine (10) for in each case a single test.

9. Process according to one of the claims 1 to 8, **characterized in that** a membrane designed to filter body fluid or a foil coated with a reactive test chemistry for an analyte in the body fluid is used as the starting material.

10. Ring magazine for a diagnostic test device in particular for examining body fluids comprising a support structure (12) and a closed membrane ring or test strip ring (22) inserted therein, which is formed from a strip material segmented by cuts (24) **characterized in that** the membrane ring or test strip ring has been produced according to one of the previous claims, and that the segments (20) form a filter or a detection element for a single test in each case.

11. Ring magazine according to claim 10, **characterized in that** the support structure (12) has a plurality of openings (18) for the transfer of body fluid and that the openings (18) are each covered by a segment (20) of the membrane ring or test strip ring (22).

12. Ring magazine according to claim 10 or 11, **characterized in that** the support structure (12) has a disc-shaped housing (12) comprising test chambers arranged in a ring shape each allocated to one segment (20).

13. Ring magazine according to one of the claims 10 to 12, **characterized in that** the segments (20) each have an application site for taking up body fluid.

14. Ring magazine according to one of the claims 10 to 13, **characterized in that** the membrane ring (22) is placed as a filter in front of a ring-shaped detection element.

15. Diagnostic test device for examining body fluids comprising a housing and a ring magazine inserted therein according to one of the claims 10 to 14.

## Revendications

1. Procédé de production d'une membrane annulaire ou d'une bandelette de test annulaire pour un dispositif de test diagnostique, en particulier pour l'analyse de liquides corporels, selon lequel :
a) une bandelette (26) allongée, fournie de préférence par découpe d'une matière de départ plane ou en forme de ruban, est subdivisée en segments (20) par des incisions (24) transversales par rapport au sens longitudinal de la bandelette,
b) les incisions (24) n'allant que jusqu'à une largeur restante de la bandelette (26), de sorte qu'un pont de matière (38) est maintenu entre les segments (20) adjacents aux incisions (24),
c) la bandelette (26) est fermée par la réunion de ses extrémités pour former une bague (22),
**caractérisé en ce que**
d) les arêtes de coupe des incisions (24) allant vers les ponts de matière (38) incluent respectivement un angle aigu (α),
e) la bague (22) étant introduite en tant que membrane annulaire ou bandelette de test annulaire pour le dispositif de test dans une structure porteuse (12), la bague (22) étant insérée dans un boîtier en forme de disque (12) en tant que structure porteuse, de sorte que les segments (20) sont respectivement associés à une chambre (14) du boîtier.

2. Procédé selon la revendication 1, **caractérisé en ce que** la bandelette (26) est incisée uniformément depuis un de ses bords longitudinaux, de sorte que les ponts de matière (38) restent sur l'autre bord longitudinal.

3. Procédé selon la revendication 1, **caractérisé en ce que** la bandelette (26) est incisée de part et d'autre sur respectivement une ligne depuis ses deux bords longitudinaux avec obtention d'un pont de matière (38).

4. Procédé selon l'une des revendications 1 à 3, **caractérisé en ce que** la bague (22) est formée de manière telle que les ponts de matière (38) forment une ligne circulaire (46), les extrémités de la bandelette (26) se contactant sur la ligne circulaire.

5. Procédé selon l'une des revendications 1 à 4, **caractérisé en ce que** les segments (20) sont, lors de la formation de la bague, posés l'un sur l'autre en chevauchement, les chevauchements (42) limités par des arêtes de coupe des incisions (24) allant du côté intérieur de la bague vers le pont de matière respectif (38).

6. Procédé selon l'une des revendications 1 à 5, **caractérisé en ce que** des segments voisins (20) sont reliés, et plus particulièrement soudés ou collés entre eux en chevauchement de préférence sous l'action de la chaleur et/ou d'une pression.

7. Procédé selon l'une des revendications 1 à 6, **caractérisé en ce que** la matière de départ est formée par une feuille revêtue d'une chimie de test réactive ou par une membrane au moins partiellement perméable au liquide corporel.

8. Procédé selon l'une des revendications 1 à 7, **caractérisé en ce que** les segments (20) sont agencés en tant que consommables dans un magasin annulaire (10) pour respectivement un test individuel.

9. Procédé selon l'une des revendications 1 à 8, **caractérisé en ce qu'**est utilisée, en tant que matière de départ, une membrane réalisée pour filtrer le liquide corporel ou une feuille revêtue d'une chimie de test réactive pour un analyte du liquide corporel.

10. Magasin annulaire pour un dispositif de test diagnostique, en particulier pour l'analyse de liquides corporels, comportant une structure porteuse (12) et une membrane annulaire ou bandelette de test annulaire fermée, introduite dedans et formée d'une bande de matière segmentée par des incisions (24), **caractérisé en ce que** la membrane annulaire ou bandelette de test annulaire (22) est réalisée selon l'une des revendications précédentes et **en ce que** les segments (20) forment un filtre ou un élément de détection pour respectivement un test individuel.

11. Magasin annulaire selon la revendication 10, **caractérisé en ce que** la structure porteuse (12) comporte une pluralité d'orifices (18) pour le transfert de liquide corporel et **en ce que** les orifices (18) sont recouverts par respectivement un segment (20) de la membrane annulaire ou bandelette de test annulaire (22).

12. Magasin annulaire selon la revendication 10 ou 11, **caractérisé en ce que** la structure porteuse (12) comporte un boîtier (12) en forme de disque avec des chambres de test agencées annulairement et respectivement associées à un segment (20).

13. Magasin annulaire selon l'une des revendications 10 à 12, **caractérisé en ce que** les segments (20) comportent respectivement un point d'application pour l'admission de liquide corporel.

14. Magasin annulaire selon l'une des revendications 10 à 13, **caractérisé en ce que** la membrane annulaire (22) est, en tant que filtre, située en amont d'un élément de détection réalisé sous forme annulaire.

15. Dispositif de test diagnostique destiné à l'analyse de liquides corporels, comportant un boîtier et un magasin annulaire inséré dedans selon l'une des revendications 10 à 14.
